# EUROPÄISCHE PATENTSCHRIFT

(11) **EP 0 350 037 B1**
(45) Veröffentlichungstag und Bekanntmachung des Hinweises auf die Patenterteilung: **16.03.1994**
(21) Anmeldenummer: 89112374.7
(22) Anmeldetag: 06.07.1989
(51) Int. Cl.: G01N 33/68, G01N 33/536, G01N 33/543, G01N 33/58

(54) **Verfahren zur Bestimmung von Antikörpern**
Method to determine antibodies
Méthode pour déterminer des anticorps

(30) Priorität: 08.07.1988 DE 3823262; 09.03.1989 DE 3907651
(43) Veröffentlichungstag der Anmeldung: 10.01.1990
(73) Patentinhaber: BOEHRINGER MANNHEIM GMBH, 68298 Mannheim (DE)
(72) Erfinder: Schmitt, Urban, D-8125 Oberhausen (DE); Rüdinger, Wolfgang, Dr. med., Dr. Ing., D-6943 Birkenau (DE); Ehrlich-Weinreich, Gertraud, Dr.phil.nat., D-8032 Gräfelfing (DE)
(74) Vertreter: Huber, Bernhard, Dipl.-Chem.

(56) Entgegenhaltungen:
- EP-A- 0 245 926
- GB-A- 2 084 317
- US-A- 4 661 444

## Beschreibung

Die Erfindung betrifft ein Verfahren zur Bestimmung von Antikörpern durch Inkubation mit mindestens drei Rezeptoren R₁ , R₂ und R₃, die in flüssiger Phase gelöst vorliegen und von denen R₁ mit dem zu bestimmenden Antikörper bindefähig ist, R₂ die Bindung an die feste Phase vermittelt und R₃ eine Markierung trägt, Abtrennung des sich bildenden Komplexes aus der Lösung durch Bindung an eine feste Phase und Messung der Markierung in einer der Phasen sowie ein hierzu geeignetes Reagenz.

Antikörper sind Proteinmoleküle, die nach Kontakt mit dem passenden Antigen von B-Lymphocyten und Plasmazellen produziert werden und spezifisch gegen das ihre Bildung auslösende Antigen gerichtet sind. Der Körper bildet Antikörper einerseits als Antwort auf das Eindringen körperfremder Moleküle wie Fremdeiweiß, Bakterien oder Viren und andererseits beim Vorliegen einer Autoimmunkrankheit auch Antikörper gegen körpereigene Zellen. Der Nachweis spezifischer Antikörper bietet daher eine Möglichkeit, den Verlauf von Krankheiten oder das Vorliegen einer Autoimmunkrankheit zu diagnostizieren.

Antikörper können empfindlich nachgewiesen werden durch Verfahren nach dem Prinzip des Immunoassays. Dazu sind eine Vielzahl von Varianten bekannt, wobei sowohl kompetitive, immunoradiometrische und immunoenzymometrische als auch Sandwichassays bekannt sind. Eine Aufzählung verschiedener Varianten ist beispielsweise in einem Artikel von A.H.W.M. Schuurs und B.K. van Weemen in Dt. Ges. f. Klin. Chemie e.V.-Mitteilungen 1/79 angegeben. Bei allen Varianten mit Ausnahme der kompetitiven Variante, muß dabei an eine Festphase eine mit dem zu bestimmenden Antikörper spezifisch reagierende Substanz gebunden werden. Dies kann entweder das mit dem zu bestimmenden Antikörper spezifisch reagierende Antigen oder Hapten sein oder aber auch ein gegen den zu bestimmenden Antikörper gerichteter Antikörper wie beispielsweise ein klassenspezifischer Antikörper. Nachteil dieser beschriebenen Verfahren ist es nun, daß für jeden Test eine spezifische Festphase zur Verfügung gestellt werden muß.

Weiterhin wird in der oben angegebenen Literaturstelle ein Testverfahren beschrieben, bei dem an eine Festphase ein gegen die Antikörperklasse, der der zu bestimmende Antikörper entstammt, gerichteter Antikörper gebunden ist. In Gegenwart dieser Festphase wird die Probelösung, die den zu bestimmenden Antikörper enthält, mit dem für ihn spezifischen Antigen sowie einem ebenfalls mit dem Antigen bindefähigen markierten Antikörper inkubiert. Dabei konkurrieren der zu bestimmende Antikörper und der zugegebene markierte Antikörper um die Bindung an das Antigen. In der homogenen Phase präformierte Immunkomplexe mit noch freien Antikörpervalenzen werden durch den festphasengebundenen Antikörper immobilisiert und nachgewiesen. Da die Kinetik der Festphasenbindung deutlich langsamer als die Kinetik der Ausbildung des Immunkomplexes in flüssiger Phase ist, verläuft die Festphasenbindung nicht nach einem kompetitiven Prinzip, sondern als Titrationsreaktion. Dieses Verfahren ist jedoch nicht ausreichend empfindlich für den Nachweis von Antikörpern, die in geringer Menge in der Probelösung vorhanden sind. Die zur Durchführung dieses Verfahrens notwendige Menge an spezifischen Antikörpern ist sehr hoch.

Aufgabe der Erfindung war es daher, ein Verfahren zur Verfügung zu stellen, mit dem Antikörper empfindlich in einem einfachen Verfahren nachgewiesen werden können, wobei der Antikörperbedarf reduziert werden kann.

Diese Aufgabe wird gelöst durch ein Verfahren zur Bestimmung von Antikörpern nach dem Prinzip des Immunoassays durch Inkubation mit mindestens drei Rezeptoren R₁, R₂ und R₃, die in flüssiger Phase gelöst vorliegen und von denen R₁ ein mit dem zu bestimmenden Antikörper spezifisch bindefähiger Anti-Idiotyp-Antikörper oder ein Antigen mit mindestens zwei für die Bindung mit dem zu bestimmenden Antikörper befähigten Epitopen ist, R₂ die Bindung an die feste Phase vermittelt und R₃ eine Markierung trägt, Abtrennung des sich bildenden Komplexes aus der Lösung durch Bindung an eine feste Phase und Messung der Markierung in einer der Phasen, das dadurch gekennzeichnet ist, daß man als Rezeptor R₂ ein Konjugat aus einem mit R₁ spezifisch bindefähigen Antikörper oder dessen Fragment und einer spezifisch bindefähigen Substanz S₁ und als R₃ ein Konjugat aus dem mit R₁ spezifisch bindefähigen Antikörper oder dessen Fragment und einer Markierung verwendet, wobei die Immobilisierung des sich bildenden Komplexes über die Bindung an eine mit S₁ spezifisch bindefähige Komponente an eine Festphase erfolgt, wobei die Rezeptoren R₂ und R₃ das vom zu bestimmenden Antikörper erkannte Epitop erkennen.

Mit dem erfindungsgemäßen Verfahren gelingt es überraschenderweise, die Empfindlichkeit bedeutend zu verbessern. Darüberhinaus ist zu seiner Durchführung eine wesentlich geringere Menge an Antikörper erforderlich.

Bei dem erfindungsgemäßen Verfahren wird die Probe, die den zu bestimmenden Antikörper enthält, mit drei Rezeptoren, die alle in flüssiger Phase gelöst vorliegen, inkubiert. Rezeptor R₁ ist dabei ein mit dem zu bestimmenden Antikörper spezifisch bindefähiger Rezeptor und kann entweder ein Antigen oder ein Anti-Idiotyp-Antikörper sein. Rezeptor R₂ ist mit R₁ spezifisch bindefähig und vermittelt die Bindung an die feste Phase, wofür er entsprechend derivatisiert ist. Rezeptor R₃ ist ebenfalls mit R₁ spezifisch bindefähig und trägt außerdem eine Markierung. In der Probelösung konkurrieren nun Rezeptor R₂ und Rezeptor R₃ zusammen mit dem zu bestimmenden Antikörper um die Bindung an Rezeptor R₁. Je mehr Antikörper nun in der Probelösung vorhanden ist, desto mehr Antikörper wird auch an R₁ gebunden und desto weniger Bindestellen stehen für die Rezeptoren R₂ und R₃ zur Verfügung. Abhängig von der Anzahl der Epitope, die das Antigen aufweist, bilden sich Komplexe von R₁ mit R₂, R₃ und/oder dem zu bestimmenden Antikörper. Nur die Komplexe, die mindestens einen Rezeptor R₂ enthalten, können an der Festphase gebunden werden und nur Komplexe, in denen mindestens ein Rezeptor R₃ gebunden ist, gehen in die Nachweisreaktion ein. Die sich bildenden Komplexe aus Rezeptor R₁, Rezeptor R₂ sowie gegebenenfalls Rezeptor R₃ und/oder dem zu bestimmenden Antikörper werden dann über die in Rezeptor R₂ gebundene spezifisch bindefähige Substanz S₁ an eine feste Phase gebunden. Dabei kann die Bindung entweder direkt über an der Festphase fixierte mit S₁ bindefähige Komponenten oder über die Bindung an die Festphase vermittelnde Komponenten erfolgen. Nach Trennung der festen von der flüssigen Phase kann die Markierung in einer der beiden Phasen bestimmt werden, wobei umso mehr gebundene Markierung nachgewiesen werden kann, je geringer der Anteil des zu bestimmenden Antikörpers in der Probelösung ist.

Der bei dem erfindungsgemäßen Verfahren verwendete Rezeptor R₁ ist ein mit dem zu bestimmenden Antikörper spezifisch bindefähiges Antigen, das mindestens zwei für die Bindung mit dem Antikörper befähigte Epitope aufweist.

Als Rezeptor R₂ wird erfindungsgemäß ein Konjugat verwendet, das aus einem mit R₁ spezifisch bindefähigen Rezeptor und einer spezifisch bindefähigen Substanz S₁ besteht. Der mit R₁ spezifisch bindefähige Anteil dient zur Bindung an das eingesetzte Antigen und ist ein Antikörper oder dessen Fragment. Die spezifisch bindefähige Substanz S₁ des Rezeptors R₂ vermittelt die Bindung an die feste Phase. Die spezifisch bindefähige Substanz S₁ ist dabei bevorzugt ein Partner eines spezifisch miteinander bindenden Paares. Derartige Paare sind dem Fachmann bekannt. Geeignet sind beispielsweise die folgenden Paare: Antigen-Antikörper; Hapten-Antikörper; Biotin-Avidin/Streptavidin; Protein-Antiprotein; Protein A-Immun-Globulin; Hämoglobin-Haptoglobin oder Enzym-Substrat. Bevorzugt wird als S₁ ein Hapten verwendet, wie beispielsweise Digoxin, p-Nitrophenol, Saponin, FITC und insbesondere Biotin. Die Bindung von S₁ an den mit R₁ spezifisch bindefähigen Rezeptor erfolgt in an sich bekannter Weise. Verfahren hierzu sind dem Fachmann bekannt.

Der dritte verwendete Rezeptor ist ein Konjugat aus einem mit R₁ spezifisch bindefähigen Rezeptor und einer Markierung. Als Markierung kann ein Enzym, eine radioaktive Substanz, ein Isotop, eine fluoreszierende oder chemilumineszierende Substanz verwendet werden, deren Bestimmung nach einer der dem Fachmann wohlbekannten Methoden durchgeführt wird. Die Herstellung der Konjugate erfolgt in an sich bekannter Weise.

Die für die Rezeptoren R₂ und R₃ verwendeten, spezifisch bindefähigen Rezeptoren sind Antikörper oder deren Fragmente, die mit dem als Rezeptor R₁ verwendeten Antigen bindefähig sind. Da diese spezifisch bindefähigen Rezeptoren mit dem zu bestimmenden Antikörper um die Bindung an das Antigen konkurrieren sollen, ist es wesentlich, daß man hier Rezeptoren verwendet, die ähnlich wie der zu bestimmende Antikörper mit dem Antigen reagieren, d.h. eine ähnliche Bindungskapazität aufweisen. Daher werden als Rezeptoren für R₂ und R₃ entweder jeweils polyklonale Antikörper verwendet, die dieselbe Bindefähigkeit wie der zu bestimmende Antikörper besitzen. Ebenso ist es möglich, einen monoklonalen Antikörper zu verwenden, wenn gesichert ist, daß das von diesem monoklonalen Antikörper erkannte Epitop am Antigen auch durch den zu bestimmenden Antikörper erkannt wird.

Die beiden Rezeptoren R₂ und R₃ müssen in einem solchen Verhältnis eingesetzt werden, daß einerseits genügend Rezeptoren R₂ an R₁ binden können, um die Immobilisierung der gebildeten Komplexe zu gewährleisten und und andererseits genügend Rezeptoren R₃ gebunden werden, da nur über die Markierung die Bestimmung erfolgen kann.

Die Immobilisierung des aus Antigen, R₂, R₃ und/oder dem zu bestimmenden Antikörper gebildeten Komplexes an eine Festphase erfolgt über die spezifisch bindefähige Substanz S₁. Hierzu gibt es zwei Varianten. In einer bevorzugten Ausführungsform sind an der Festphase mit S₁ spezifisch bindefähige Komponenten gebunden. Hierzu werden die zu S₁ komplementären Partner des spezifisch bindenden Paares an eine feste Phase in an sich bekannter Weise gebunden. Die kompetitive Reaktion der Rezeptoren R₂, R₃ und des zu bestimmenden Antikörpers mit R₁ wird nicht gestört, auch wenn die Inkubation in Gegenwart der Festphase erfolgt, da die Reaktion der Rezeptoren R₁, R₂ und R₃ in homogener Phase stattfindet und daher wesentlich schneller abläuft als die Bindung an die heterogene Phase. Ebenso möglich ist es natürlich, zuerst die Komplexbildung durchzuführen und dann die feste Phase zuzugeben, nach deren Zugabe die Wandbindung erst erfolgen kann. Als feste Phase sind sowohl Polymermaterialien als auch cellulosehaltige Materialien oder Glas geeignet. Als besonders geeignet haben sich Polystyrol, Polymethacrylat, Teflon, Polyamid, Copolymere aus Styrol und Acrylnitril, Glas und Celluloseprodukte erwiesen. Die feste Phase kann in beliebiger Form vorliegen, z. B. als Röhrchen, Mikrotiterplatten, Kugeln, Film, Pulver, Körnchen oder Faservlies.

Die spezifisch bindefähige Komponente kann in an sich bekannter Weise an die Festphase gebunden werden. Die Bindung kann dabei entweder direkt an die Festphase erfolgen oder über einen Spacer oder ein Bindeprotein. Verfahren hierzu sind dem Fachmann bekannt. Geeignet ist beispielsweise ein in der Patentanmeldung DE-A 36 40 412 beschriebenes Verfahren zur Herstellung einer Festphasenmatrix.

In einer weiteren bevorzugten Ausführungsform des erfindungsgemäßen Verfahrens wird an der festen Phase, eine zweite spezifisch bindefähige Substanz S₁ gebunden. Diese Substanz S₂ ist ebenso wie die Substanz S₁ Partner eines spezifisch bindenden Paares, wie es oben definiert ist. Besonders bevorzugt sind S₁ und S₂ identisch. Zur Immobilisierung wird dann eine Komponente zugegeben, die mindestens je eine spezifische Bindungsstelle für S₁ und S₂ aufweist. Diese Komponente kann ein Molekül sein, das zwei oder mehr Bindungsstellen für S₁ bzw. S₂ aufweist, sie kann ebenso auch ein Konjugat aus zwei verschiedenen Molekülen sein, die jeweils eine spezifische Bindungsstelle für S₁ bzw. S₂ aufweisen. Weiterhin ist es möglich, die Komponente in vernetzter Form einzusetzen, d. h. Polymere von mit S₁ bzw. S₂ bindefähigen Substanzen.

Besonders bevorzugt wird als S₁ und S₂ jeweils Biotin verwendet. Zur Immobilisierung kann dann Avidin oder Streptavidin verwendet werden, das entweder in monomerer, aber auch in polymerer Form vorliegen kann.

Durch Zugabe der Komponente zu der Lösung wird das gebildete Konjugat aus Antigen und R₂, R₃ und/oder zu bestimmendem Antikörper durch die Bindung der Komponente mit S₁ und die Bindung der Komponente mit S₂ an der Festphase fixiert. Die Phasen können dann leicht getrennt werden, indem die Flüssigkeit entfernt wird. Nach Trennung der Phasen kann dann die Markierung in einer der beiden Phasen in an sich bekannter Weise bestimmt werden und ist dann ein Maß für die Menge an antigenspezifischem Antikörper.

Das erfindungsgemäße Verfahren ist einfach durchzuführen, da es in einer oder höchstens zwei Stufen durchgeführt werden kann. Damit besteht auch die Möglichkeit, dieses Verfahren auf einen Analyseautomaten zu übertragen. Da das Verfahren kompetitiv abläuft, kann gegenüber den bisher bekannten und meist verwendeten Bestimmungsverfahren, die alle als Sandwich-Verfahren oder immunometrische Verfahren ausgebildet sind, die Menge an benötigtem Antikörper wesentlich reduziert werden. Trotzdem werden mit dem erfindungsgemäßen Verfahren sehr gute Ergebnisse erhalten, wobei die Empfindlichkeit gegenüber den bekannten Verfahren noch weiter verbessert werden konnte.

Ein weiterer Gegenstand der vorliegenden Erfindung ist ein Reagenz zur Bestimmung von Antikörpern, das Rezeptoren R₁, R₂ und R₃ sowie eine Festphase physikalisch getrennt voneinander enthält, wobei R₁ ein mit dem zu bestimmenden Antikörper spezifisch bindefähiger Anti-Idiotyp-Antikörper oder ein Antigen mit mindestens 2 für die Bindung mit dem zu bestimmenden Antikörper befähigten Epitopen ist, R₂ ein Konjugat aus einem mit R₁ spezifisch bindefähigen Antikörper oder dessen Fragment und einer spezifisch bindefähigen Substanz S₁ ist und R₃ ein Konjugat aus einem mit R₁ spezifisch bindefähigen Antikörper oder dessen Fragment und einer Markierung ist.

Bevorzugt wird für das erfindungsgemäße Reagenz eine Festphase verwendet, an der eine mit S₁ spezifisch bindefähige Komponente gebunden ist. Besonders bevorzugt erfolgt die Immobilisierung über das spezifisch bindende Paar Biotin-Avidin/Streptavidin.

Ebenso bevorzugt ist es, eine Festphase zu verwenden, an der eine zweite spezifisch bindefähige Substanz S₂ gebunden ist, wobei dann das Reagenz zusätzlich eine Komponente enthält, die mindestens je eine spezifische Bindungsstelle für S₁ und S₂ aufweist. Bevorzugt wird an die feste Phase dieselbe spezifisch bindefähige Substanz gebunden, wie sie im Rezeptor R₂ gebunden ist. Auch bei dieser Ausführungsform wird besonders bevorzugt als Bindungspaar Biotin und Avidin/Streptavidin verwendet.

Die Erfindung wird durch die folgenden Figuren und Beispiele erläutert.
- Fig. 1: zeigt eine Eichkurve für eine Bestimmung von Antikörpern gegen HBcAG nach dem erfindungsgemäßen Verfahren
- Fig.2: zeigt eine Eichkurve für eine Bestimmung von Antikörpern gegen HBcAG nach dem Stand der Technik

Der in den Beispielen genannte monoklonale Antikörper gegen HBcAg ist bei der European Collection of Animal Cell Cultures unter folgender Bezeichnung hinterlegt: ECACC 88022507.

### Beispiel 1

Es wurden Rezeptoren R₂ und R₃ für die Bestimmung von Antikörpern gegen HBcAg hergestellt.
a) Herstellung eines Konjugats eines monoklonalen Antikörpers gegen HBcAg (Hepatitis B-Core-Antigen) und Biotin.
   6 mg/ml D-Biotin-ε-aminocapronsäure-N-hydroxysuccinimidester wurden in Dimethylsulfoxid vorgelöst. Anschließend wurden 10 mg/ml Antikörper in 30 mmol/l Kaliumphosphatpuffer, pH 7,5, mit einem 15fachen molaren Überschuß an Biotinylierungsreagenz eine Stunde bei 25°C inkubiert. Anschließend wurde gegen 2 mmol/l Kaliumphosphatpuffer, pH 7,5, dialysiert.
b) Konjugat aus einem monoklonalen Antikörper gegen HBcAg (ECACC 88022507) und Peroxidase
   Meerrettich-Peroxidase wurde gemäß der von Nakane beschriebenen Perjodat-Methode oxidiert (Wilson, M.B., Nakane, P.K. "Immunfluorescence and Related Staining Techniques" W. Knapp, Holuber und G. Wick, Herausgeber Elsevier/North-Holland Biomedical Press Amsterdam New York 1978, Seiten 215 bis 224). 10 mg Antikörper und 12 mg oxidierte Peroxidase wurden dann in einem Gesamtvolumen von 4,6 ml bei einem pH von 9,4 und 25°C miteinander gekoppelt. Die Vernetzung wurde nach einer Stunde bei 0°C durch Zugabe von 20 mmol/l Triethanolamin und 1,5 mmol/l Natriumborhydrid durch 30minütige Inkubation bei pH 8,9 gestoppt. Das Konjugat wurde anschließend noch einer Gelfiltration an Superose 6 prep grade in 50 mmol/l Kaliumphosphatpuffer, pH 7,5 und 150 mmol/l NaCl fraktioniert.

### Beispiel 2

Es wurde in menschlichem Serum der Gehalt an Antikörpern gegen Hepatitis B-Core-Antigen bestimmt. Dazu wurden die folgenden Reagenzien verwendet:
- Reagenz 1:: 100 ng/ml rekombiniertes HBcAg (hergestellt nach EP-A 0013828)
- Reagenz 2:: 50 ng/ml biotinylierter monoklonale Antikörper gegen HBcAg (ECACC 88022507), hergestellt nach Beispiel 1a) und Konjugat aus monokolonalem Antikörper gegen HBcAg und Peroxidase (Herstellung nach Beispiel 1b), POD-Akivität 25 mU/ml) in 40 mmol/l Natriumphosphatpuffer, pH 7,4
0,5 % Polyetherglycol (Pluronic F-68)
0,2 % Rinderserumalbumin
0,1 % Rinder IgG
0,2 mol/l Natriumtartrat.

In ein mit Streptavidin-Thermo-Rinderserumalbumin beschichtetes Polystyrol-Tube, das wie in DE-A 36 40 412 beschrieben, hergestellt war, wurden 200 µl Probe pipettiert und kurz hintereinander bzw. gleichzeitig je 500 µl Reagenz 1 und Reagenz 2 zugegeben. Nach einer Stunde Inkubation bei Raumtemperatur wurde dreimal mit Leitungswasser gewaschen und 1 ml ABTS (2,2′-Azino-Di-[3-ethyl-benzthiazolin-sulfonsäure(6)]-diammoniumsalz) (1,9 mmol/l) für die Substratreaktion zupipettiert und eine weitere Stunde bei Raumtemperatur inkubiert. Die Extinktion wurde bei 405 nm in einer Küvette mit 5 mm Schichtdicke gemessen und auf 1 cm-Küvette umgerechnet.

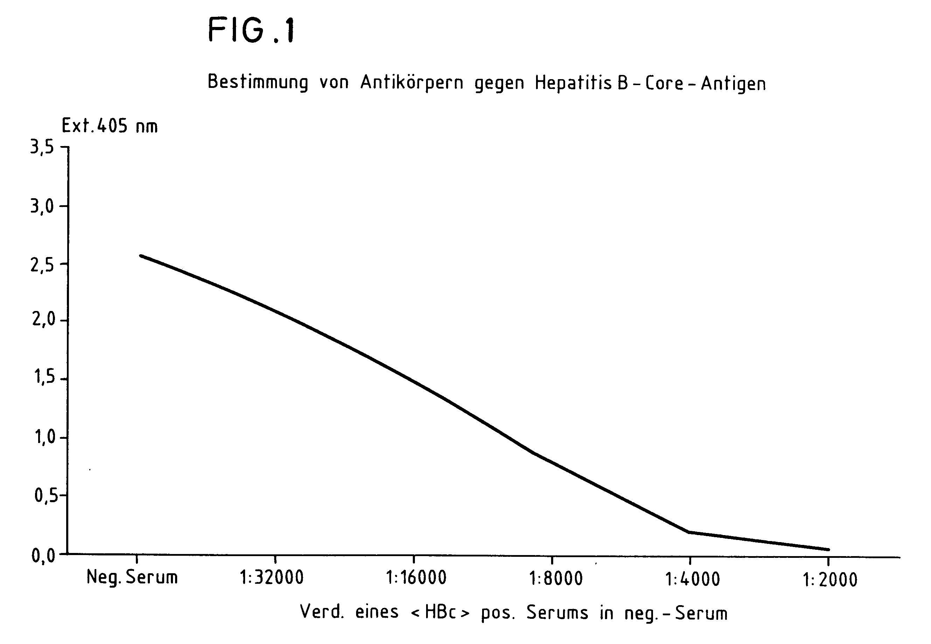

- Fig. 1: zeigt eine Eichkurve, die durch Verdünnung eines positiven Serums in ein negatives erhalten wurde. In Tabelle 1 sind die Meßwerte zusammengefaßt.

**Tabelle 1**

| Verd. HBc | Beispiel 2 | Beispiel 3 |
|---|---|---|
| | Ext._{405 nm} | Ext._{405 nm} |
| 1 : 2000 | 0,048 | 0,040 |
| 1 : 4000 | 0,195 | 0,803 |
| 1 : 8000 | 0,792 | 1,856 |
| 1 : 16000 | 1,489 | 2,565 |
| 1 : 32000 | 2,070 | 2,767 |
| Neg. Serum | 2,570 | 2,845 |
| Nachweisgrenze | 1,050 | 1,225 |

### Beispiel 3 (Vergleich)

Zum Vergleich wurde eine Bestimmung von Antikörpern gegen HBcAg, wie sie aus dem Stand der Technik bekannt ist, durchgeführt. Dazu wurden Polystyrol-Tubes mit 1,5 ml einer Lösung gefüllt, die aus 3 µg/ml monoklonalem Antikörper gegen HBcAg (ECACC 88022507) in 40 mmol/l Phosphatpuffer, pH 7,4, enthielt und 24 Stunden bei Raumtemperatur stehen gelassen. Nach Entfernen der Lösung wurden die Röhrchen mit 2 ml Puffer, bestehend aus 0,9 % NaCl, 0,3 % Rinderserumalbumin und 2 % Saccharose gefüllt und eine halbe Stunde inkubiert. Nach Entfernung dieser Lösungen wurden die Röhrchen mit der Öffnung nach unten über Nacht bei 21°C inkubiert. Es wurde weiter, wie im Beispiel 2 beschrieben, verfahren, wobei anstelle der mit Streptavidin-Thermo-RSA beschichteten Polystyrol-Tubes ein mit monoklonalen Antikörpern gegen HBcAg beschichtetes Polystyrol-Tube verwendet wurde und Reagenz 2 ohne biotinylierten monoklonalen Antikörper gegen HBcAg hergestellt.

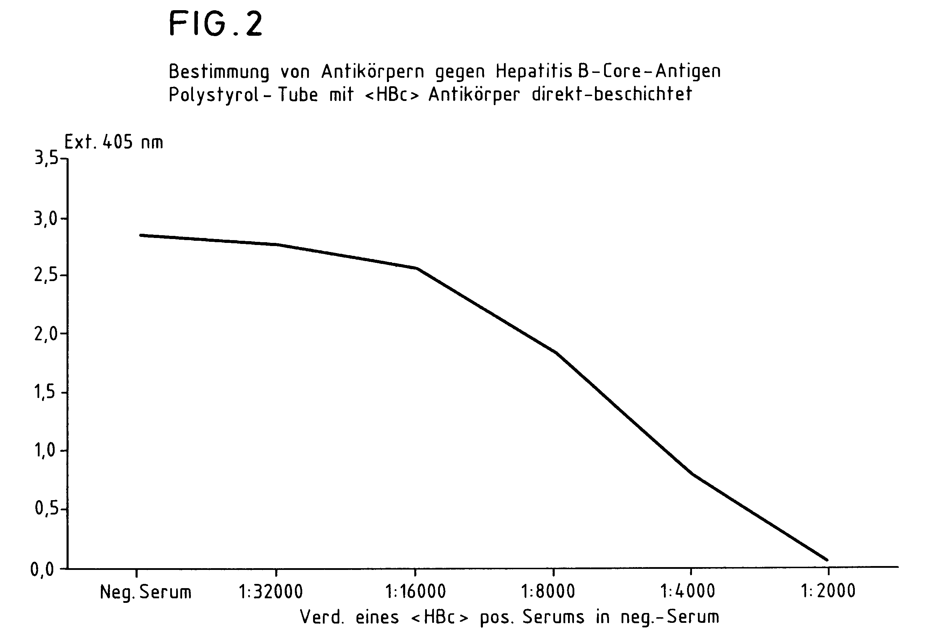

Die Ergebnisse sind in Tabelle 1 angegeben. Fig. 2 zeigt eine Eichkurve, die durch Verdünnen eines positiven Serums in ein negatives Serum erhalten wurde.

### Beispiel 4

Zur Bestimmung von Antikörpern gegen Hepatitis B-Core-Antigen in Serum wurde ein kompetitiver Test durchgeführt. Dazu wurde in ein mit Streptavidin-Thermo-Rinderserumalbumin beschichtetes Polystyrol (hergestellt wie in Beispiel 2) 500 µl eines Reagenzes, bestehend aus:
- 50 ng/ml: biotinylierten monoklonalen Antikörpern gegen HBcAg (ECACC 88022507), die gemäß Beispiel 1a) hergestellt wurden (Rezeptor R₂),
- 100 ng/ml: rekombiniertes HBcAg, das gemäß der in EP-A 0013828 beschriebenen Vorschrift hergestellt wurde (Rezeptor R₂) und
- 200 µl: Probe
in eine Lösung inkubiert, die 40 mmol/l Natriumphosphatpuffer, pH 7,4, 0,5 % Polyetherglykol (Pluronic F-68), 0,2 % Rinderserumalbumin, 0,1 % Rinder-IgG, 0,2 mol/l Natriumtartrat enthielt, pipettiert. Nach einer Stunde Inkubation bei Raumtemperatur wurden 500 µl eines zweiten Reagenzes, das das Konjugat von monoklonalem Antikörper gegen HBcAg und Peroxidase, das gemäß Beispiel 1b) erhalten wurde (Rezeptor R₃) mit einer Peroxidase-Aktivität von 25 mU/ml in die oben beschriebene Pufferlösung zugegeben und eine weitere Stunde bei Raumtemperatur inkubiert. Nach erfolgter Inkubation wurde dreimal mit Leitungswasser gewaschen und 1 ml ABTS (1,9 mmol/l) für die Substratreaktion zupipettiert und eine Stunde bei Raumtemperatur inkubiert. Die Extinktion wurde bei 405 nm in einer Küvette mit 5 mm Schichtdicke gemessen und auf eine 1-cm-Küvette umgerechnet.

Die Meßwerte sind in der Tabelle 2 zusammengefaßt.

### Beispiel 5

Zur Bestimmung von Antikörpern gegen Hepatitis-B-Core-Antigen nach dem Kompetitionsprinzip wurden in ein mit Streptavidin-Thermo-Rinderserumalbumin beschichtetes Polystyrol-Tube, hergestellt wie in DE-A 3640412 beschrieben, 200 µl Probe und 500 µl Reagenz 1, bestehend aus 100 ng/ml rekombiniertem HBcAg (hergestellt nach EP 0013828) in 40 mmol/l Natriumphosphatpuffer, pH 7,4, 0,5 % Polyetherglykol (Pluronic F 68), 0,2 % Rinderserumalbumin, 0,1 % Rinder IgG, 0,2 mol/l Natriumtartrat pipettiert. Nach 1 h Inkubation wurden 500 µl Reagenz 2 bestehend aus
- 50 ng/ml: biotinylierten monoklonalen Antikörpern gegen HBcAg, die (gemäß Beispiel 1a) hergestellt wurden und
- 25 mU/ml: Konjugat aus monoklonalen Antikörpern gegen HBcAg und Peroxidase (hergestellt wie in Beispiel 1b) beschrieben
in einer Pufferlösung wie oben bei Reagenz 1 angegeben, zugegeben und 1 weitere Stunde bei Raumtemperatur inkubiert.

Weiter wurde, wie in Beispiel 3 beschrieben, verfahren.

Die erhaltenen Meßwerte sind in Tabelle 2 zusammengefaßt.

**Tabelle 2**

| Anti-HBc Positiv-Serum Verdünnung | Beispiel 4 Ext. 405 nm | Beispiel 5 Ext. 405 nm |
|---|---|---|
| 1 : 2000 | 0,075 | 0,016 |
| 1 : 4000 | 0,163 | 0,046 |
| 1 : 8000 | 0,357 | 0,229 |
| 1 : 16000 | 0,637 | 0,563 |
| 1 : 32000 | 0,907 | 0,814 |
| Neg. Serum | 1,165 | 1,206 |
| Nachweisgrenze | 0,491 | 0,493 |

## Patentansprüche

1. Verfahren zur Bestimmung von Antikörpern nach dem Prinzip des Immunoassays durch Inkubation mit mindestens drei Rezeptoren R₁, R₂ und R₃, die in flüssiger Phase gelöst vorliegen und von denen R₁ ein mit dem zu bestimmenden Antikörper spezifisch bindefähiger Anti-Idiotyp-Antikörper oder ein Antigen mit mindestens zwei für die Bindung mit den zu bestimmenden Antikörper befähigten Epitopen ist, R₂ die Bindung an die feste Phase vermittelt und R₃ eine Markierung trägt, Abtrennung des sich bildenden Komplexes aus der Lösung durch Bindung an eine feste Phase und Messung der Markierung in einer der Phasen,
**dadurch gekennzeichnet**,
daß man als Rezeptor R₂ ein Konjugat aus einem mit R₁ spezifisch bindefähigen Antikörper oder dessen Fragment und einer spezifisch bindefähigen Substanz S₁ und als R₃ ein Konjugat aus dem mit R₁ spezifisch bindefähigen Antikörper oder dessen Fragment und einer Markierung verwendet, wobei die Immobilisierung des sich bildenden Komplexes über die Bindung an eine mit S₁ spezifisch bindefähige Komponente an eine Festphase erfolgt, wobei die Rezeptoren R₂ und R₃ das vom zu bestimmenden Antikörper erkannte Epitop erkennen.

2. Verfahren nach Anspruch 1,
**dadurch gekennzeichnet,**
daß man den zu bestimmenden Antikörper gleichzeitig mit allen drei Rezeptoren versetzt.

3. Verfahren nach Anspruch 1,
**dadurch gekennzeichnet,**
daß man den zu bestimmenden Antikörper zuerst mit Rezeptor R₁ und gegebenenfalls R₂ oder R₃ inkubiert und dann der Lösung die Rezeptoren R₂ und R₃ bzw. nur R₃ oder R₂ zusetzt.

4. Verfahren nach Anspruch 1, 2 oder 3,
**dadurch gekennzeichnet,**
daß man eine feste Phase verwendet, an der spezifisch mit S₁ bindefähige Komponenten gebunden sind.

5. Verfahren nach Anspruch 1, 2 oder 3,
**dadurch gekennzeichnet,**
daß man eine Festphase verwendet, an der eine zweite spezifisch bindefähige Substanz S₂ gebunden ist und daß man nach Inkubation der Probelösung mit den an der Reaktion beteiligten Rezeptoren zur Immobilisierung des Komplexes eine Komponente zugibt, die mindestens je eine spezifische Bindungsstelle für S₁ und S₂ aufweist.

6. Verfahren nach einem der vorhergehenden Ansprüche,
**dadurch gekennzeichnet,**
daß man für R₂ und R₃ Rezeptoren verwendet, die dieselbe Bindefähigkeit aufweisen wie der zu bestimmende Antikörper.

7. Verfahren nach einem der vorhergehenden Ansprüche,
**dadurch gekennzeichnet,**
daß man für R₂ und R₃ als Rezeptoren polyklonale Antikörper verwendet, die mit R₁ bindefähig sind.

8. Verfahren nach einem der vorhergehenden Ansprüche,
**dadurch gekennzeichnet,**
daß man als spezifisch bindendes Paar S₁ - Komponente eines der Paare Antigen-Antikörper; Hapten-Antikörper; Protein-Antiprotein, Protein A-Immun-Globulin; Hämoglobin-Haptoglobin; Enzym-Substrat oder Avidin/Streptavidin-Biotin verwendet wird.

9. Reagenz zur Bestimmung von Antikörpern nach dem Prinzip des Immunoassays nach einem der Ansprüche 1-8,
**dadurch gekennzeichnet**,
daß es drei Rezeptoren R₁, R₂ und R₃, sowie eine Festphase physikalisch getrennt voneinander enthält, wobei R₁ ein mit dem zu bestimmenden Antikörper spezifisch bindefähiger Anti-Idiotyp-Antikörper oder ein Antigen mit mindestens 2 für die Bindung mit dem zu bestimmenden Antikörper befähigten Epitopen ist, R₂ ein Konjugat aus einem mit R₁ spezifisch bindefähigen Antikörper oder dessen Fragment und einer spezifisch bindefähigen Substanz S₁ ist und R₃ ein Konjugat aus einem mit R₁ spezifisch bindefähigen Antikörper oder dessen Fragment und einer Markierung ist.

10. Reagenz nach Anspruch 9,
**dadurch gekennzeichnet,**
daß es eine Festphase enthält, an der eine zweite spezifisch bindefähige Substanz S₂ gebunden ist und daß das Reagenz weiterhin eine Komponente enthält, die mindestens je eine spezifische Bindungsstelle für S₁ und S₂ aufweist.

## Claims

1. Method for the determination of antibodies based on an immunoassay technique by incubation with at least three receptors R₁, R₂ and R₃ which are present dissolved in a liquid phase and of which R₁ is an anti-idiotype antibody which is capable of specific binding to the antibody to be determined or an antigen with at least two epitopes which enable binding to the antibody to be determined, R₂ mediates the binding to the solid phase and R₃ carries a label, separation of the complex which forms from the solution by binding to a solid phase and measurement of the label in one of the phases,
**wherein**
a conjugate composed of an antibody capable of specific binding to R₁ or its fragment and a specifically bindable substance S₁ is used as the receptor R₂ and a conjugate of the antibody capable of specific binding to R₁ or its fragment and a label is used as R₃, whereby the immobilization of the complex which forms is achieved by binding to a component of the solid phase which can specifically bind to S₁, wherein receptors R₂ and R₃ recognize the epitope which is recognized by the antibody to be determined.

2. Method as claimed in claim 1,
**wherein**
all three receptors are added simultaneously to the antibody to be determined.

3. Method as claimed in claim 1,
**wherein**
the antibody to be determined is firstly incubated with receptor R₁ and, if desired, with R₂ or R₃ and then the receptors R₂ and R₃ or only R₃ or R₂ are added to the solution.

4. Method as claimed in claim 1, 2 or 3,
**wherein**
a solid phase is used on which components are bound which are capable of specific binding to S₁.

5. Method as claimed in claim 1, 2 or 3,
**wherein**
a solid phase is used on which a second specifically bindable substance S₂ is bound and, after incubation of the sample solution with the receptors which take part in the reaction, a component is added to immobilize the complex which has at least one specific binding site for each of S₁ and S₂.

6. Method as claimed in one of the previous claims,
**wherein**
receptors are used for R₂ and R₃ which have the same binding capability as the antibody to be determined.

7. Method as claimed in one of the previous claims,
**wherein**
polyclonal antibodies are used as the receptors R₂ and R₃ which are capable of binding to R₁.

8. Method as claimed in one of the previous claims,
**wherein**
one of the pairs antigen-antibody; hapten-antibody; protein-antiprotein; protein A-immunoglobulin; haemoglobin-haptoglobin; enzyme-substrate or avidin/streptavidin-biotin is used as the specific binding pair S₁ - component.

9. Reagent for the determination of antibodies based on an immunoassay technique as claimed in one of the claims 1 to 8,
**wherein**
it contains three receptors R₁, R₂ and R₃ as well as a solid phase which are physically separated from one another, wherein R₁ is an anti-idiotype antibody which is capable of specific binding to the antibody to be determined or an antigen with at least two epitopes which enable binding to the antibody to be determined, R₂ is a conjugate of a receptor capable of specific binding to R₁ or its fragment and a specifically bindable substance S₁ and R₃ is a conjugate of a receptor capable of specific binding to R₁ or its fragment and a label.

10. Reagent as claimed in claim 9,
**wherein**
it contains a solid phase on which a second specifically bindable substance S₂ is bound and in addition contains a component which has at least one specific binding site for each of S₁ and S₂.

## Revendications

1. Procédé de détermination d'anticorps selon le principe de l'immuno-analyse, par incubation avec au moins trois récepteurs R₁, R₂ et R₃, qui sont présents sous forme dissoute en phase liquide et parmi lesquels R₁ est un anticorps anti-idiotype capable de se lier spécifiquement avec l'anticorps à déterminer ou un antigène comportant au moins deux épitopes capables de se lier avec l'anticorps à déterminer, R₂ permet la liaison avec la phase solide et R₃ porte un marqueur, séparation du complexe qui se forme à partir de la solution par liaison à une phase solide et mesure du marqueur dans l'une des phases, caractérisé en ce que l'on utilise comme récepteur R₂ un conjugué d'un anticorps capable de se lier spécifiquement avec R₁ ou de son fragment et d'une substance capable de se lier spécifiquement S₁ et comme R₃ un conjugué de l'anticorps capable de se lier spécifiquement avec R₁ ou de son fragment et d'un marqueur, l'immobilisation du complexe qui se forme se faisant sur une phase solide par l'intermédiaire de la liaison avec un constituant capable de se lier spécifiquement avec S₁, les récepteurs R₂ et R₃ reconnaissant l'épitope reconnu par l'anticorps à déterminer.

2. Procédé selon la revendication 1, caractérisé en ce que l'on ajoute simultanément les trois récepteurs aux anticorps à déterminer.

3. Procédé selon la revendication 1, caractérisé en ce que l'on incube l'anticorps à déterminer tout d'abord avec le récepteur R₁ et éventuellement R₂ ou R₃ puis on ajoute à la solution les récepteurs R₂ et R₃ ou seulement R₃ ou R₂.

4. Procédé selon la revendication 1, 2 ou 3, caractérisé en ce que l'on utilise une phase solide à laquelle sont liés des constituants capables de se lier spécifiquement à S₁.

5. Procédé selon la revendication 1, 2 ou 3, caractérisé en ce que l'on utilise une phase solide à laquelle est liée une seconde substance capable de se lier spécifiquement S₂ et en ce que, après l'incubation de la solution échantillon avec les récepteurs participant à la réaction pour l'immobilisation du complexe, on ajoute un constituant qui présente au moins un site de liaison spécifique pour S₁ et un site de liaison spécifique pour S₂.

6. Procédé selon l'une des revendications précédentes, caractérisé en ce que l'on utilise pour R₂ et R₃ des récepteurs qui présentent la même capacité de liaison que l'anticorps à déterminer.

7. Procédé selon l'une des revendications précédentes, caractérisé en ce que l'on utilise pour R₂ et R₃ comme récepteurs des anticorps polyclonaux qui sont capables de se lier avec R₁.

8. Procédé selon l'une des revendications précédentes, caractérisé en ce que l'on utilise comme paire se liant spécifiquement S₁-constituant l'une des paires antigène-anticorps, haptène-anticorps, protéine-antiprotéine, protéine A-immunoglobuline, hémoglobine-haptoglobine, enzyme-substrat ou avidine/streptavidine-biotine.

9. Réactif de détermination d'anticorps selon le principe de l'immuno-analyse selon l'une des revendications 1 à 8, caractérisé en ce qu'il contient physiquement séparés les uns des autres trois récepteurs R₁, R₂ et R₃ ainsi qu'une phase solide, R₁ étant un anticorps anti-idiotype capable de se lier spécifiquement avec l'anticorps à déterminer ou un antigène comportant au moins deux épitopes capables de se lier avec l'anticorps à déterminer, R₂ étant un conjugué d'un anticorps capable de se lier spécifiquement avec R₁ ou de son fragment et d'une substance capable de se lier spécifiquement S₁ et R₃ étant un conjugué d'un anticorps capable de se lier spécifiquement avec R₁ ou de son fragment et d'un marqueur.

10. Réactif selon la revendication 9, caractérisé en ce qu'il contient une phase solide à laquelle est liée une seconde substance capable de se lier spécifiquement S₂ et en ce que le réactif contient en outre un constituant qui présente au moins un site de liaison spécifique pour S₁ et un site de liaison spécifique pour S₂.
